# EUROPEAN PATENT APPLICATION

(11) **EP 2 700 641 A1**
(43) Date of publication of application: **26.02.2014**
(21) Application number: 12181409.9
(22) Date of filing: 22.08.2012
(51) Int. Cl.: C07D 487/22, H01G 9/20, H01L 51/00

(54) **Azulenocyanine analogue compounds comprising anchoring group, method of making the same, and their use as dye for dye-sensitized solar cells**

(71) Applicant: Solvay SA, 1120 Bruxelles (BE)
(72) Inventor: Braun, Max Josef, 309000 Wedemark (DE); Gutmann, Sebastian, 30625 Hannover (DE); Woehrle, Dieter, 28359 Bremen (DE); Miyaji, Taichi, Zama-shi, Kanagawa-ken, Kanagawa 252-0004 (JP)
(74) Representative: Mross, Stefan P.M.

(57) **Abstract**

Disclosed are azulenocyanine analogue compounds derived from azulene, method of making the same, and their use as dye for dye-sensitized solar cell. The azulenocyanine analogue compounds according to the present invention comprise at least one anchoring group.

## Description

### TECHNICAL FIELD

The present invention relates to azulenocyanine analogue macrocyclic compounds comprising at least one anchoring group, method(s) of making them, and their use as dyes in photoelectric conversion devices, in particular in dye-sensitized solar cells (DSSC) or in solid-state organic photovoltaics (OPV).

### BACKGROUND OF THE INVENTION

Conventional solar cells convert light into electricity by exploiting the photovoltaic effect that exists at semiconductor junctions. In other words, the commercial solar cells absorb energy from visible light and convert excited charge carriers thereof to electric energy. At present, the main commercial solar cells are silicon-based solar cells. For the silicon-based solar cell, there are shortcomings in that high energy costs for material processing is required and many problems to be addressed such as environmental burdens and cost and material supply limitations are involved. For an amorphous silicon solar cell, there are also shortcomings in that energy conversion efficiency decreases when used for a long time, due to deterioration in a short period.

One of the low-cost organic solar cells is a dye-sensitized solar cell (DSSC) which is based on a semiconductor formed based on a semiconductor sensitized electrode, a counter electrode and an electrolyte, and generally uses an organic dye to absorb incoming light to produce excited electrons. Macrocyclic compounds, one of the organic dyes in this connection, especially porphyrin derivatives and recently also phthalocyanine derivatives are subject of interest for this type of sensitization dyes in DSSC. They can exhibit conversion efficiencies up to around 12 % and 5.5 %, respectively (A. Yella, Md. Nazeeruddin, M. Grätzel et al, Science 2011, 334, 629-634 ; M-E. Ragoussi, Md. Nazeeruddin, M. Grätzel et al, Angew. Chem. Int. Ed. 2012, 51, 4375-4378). The disadvantage of porphyrins and phthalocyanines in organic solar cells is up to now that they absorb only visible light up to around 750 nm.

Japanese Patent Application Kokai JP 2011-116717A discloses a compound called azulenocyanine which is a condensation product of certain azulene rings having a formula (1) below and its isomer, which absorbs a near-infrared-light : 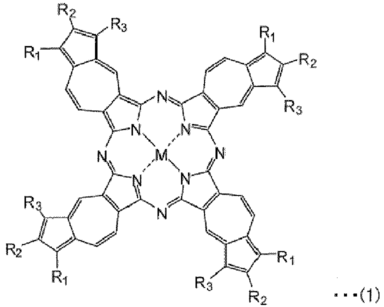 wherein R1, R2 and R3 are independently a hydrogen atom, an aromatic ring group which may have a substituent, or a non-aromatic ring substituent having 0-20 carbon, where the R1 and R2, R2 and R3 existing in the identical azulene ring may form a ring unitedly, respectively ; and M is two hydrogen atoms or the cation atom is at least bivalent ; in case said cation atom is at least trivalent, M may further comprise a counter-anion having no more than 20 carbons.

### DESCRIPTION OF THE INVENTION

The purpose of the present invention is to provide novel azulenocyanine analogue compound dyes having advantageous performance(s) when used in photoelectric conversion devices, especially in dye sensitized solar cells (DSSC) or in solid-state organic photovoltaics (OPV).

The present invention therefore relates to azulenocyanine analogue compounds comprising at least one anchoring group. It has been surprisingly found that the azulenocyanine analogue compounds according to the present invention provide improved characteristics, for example a high molar extinction coefficient, and therefore, high conversion efficiencies. Also, it has been found that a wider-range of light absorption, for instance the absorptions from the visible region of light to the near-IR (NIR) region, when used in photoelectric conversion devices, such as in DSSC or in solid-state OPV, can be attained by the azulenocyanine analogue compounds according to the present invention.

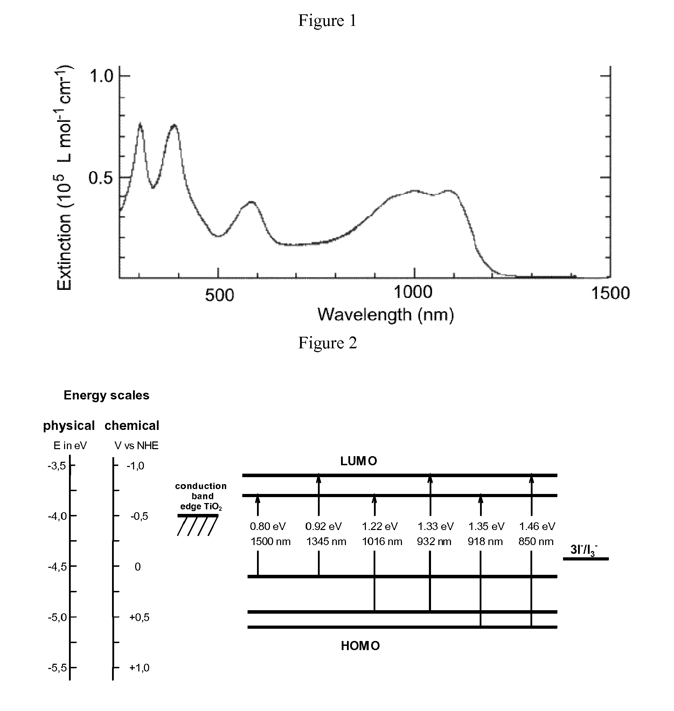

The azulenocyanine analogue compounds according to the present invention, when used especially in DSSC, can also exhibit a very high electron injection speed into a semiconductor over the wide-range of solar radiation including whole visible region and near-IR regions (see Figure 1), noting that about 48 % of the solar radiation is in the visible region and about 52 % in the near-IR region. Also, the azulenocyanine analogue compounds of the present invention can show an improved communication and directionality of the electrons when being transferred from the sensitizer to the semiconductor electrode. HOMO/LUMO calculations confirmed that photoinduced electron transfer from the exited state to TiO₂ is efficiently possible (Figure 2). Such azulenocyanine analogue compounds can further contribute to the long-term stability of such devices, for example, better resistance to water contained in trace amounts in the devices and better shielding of the electrode against corrosion through components present in the electrolyte, such as the triiodide/iodide or cobalt complex couples. Additionally, the azulenocyanine analogue compounds according to the present invention can be anchored and/or persistently attached to the semiconductor surface and/or to the surface of the semiconductor electrode.

In the present invention, "azulenocyanine analogue" is understood to denote in particular a macrocyclic molecule different from azulenocyanine which at least comprising one optionally substituted azulene ring structure. In particular, in the sense of the present invention, azulenocyanine analogue comprises at least one sub-unit of azulenocyanine structure as a part of its macrocyclic structure, of which the sub-unit can be represented by the structures below : 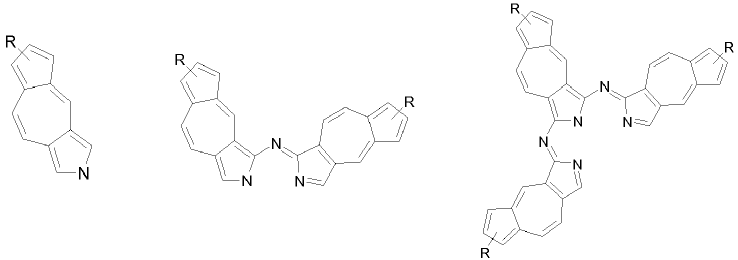 wherein R is independently selected from the group consisting of hydrogen, hydroxyl, nitro, cyano, halogens, such as fluoro, alkyl groups, halogenated alkyl groups, such as fluorinated alkyl groups, alkoxy groups, halogenated alkoxy groups, such as fluorinated alkoxy groups, aryl group, halogenated aryl groups, such as fluorinated aryl groups, aryloxy groups, halogenated aryloxy groups, such as fluorinated aryloxy groups, -OC-R' (wherein R' is selected from hydrogen, alkyl group, halogenated alkyl groups, such as fluorinated alkyl groups, aryl groups, and halogenated aryl groups, such as fluorinated aryl groups), -CH₂-R" (wherein R" is selected from the group consisting of alkoxy groups, halogenated alkoxy groups, such as fluorinated alkoxy groups, aryoxy groups, and halogenated aryloxy groups, such as fluorinated aryloxy groups, and -NR'₂), -N=N-R', -COH(R')₂, -CH=CH-R', and -C≡C-R';

In the present invention, "azulene" is understood to denote in particular an organic compound comprising a structure below :

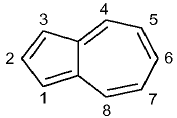

In the present invention, "alkyl groups" is understood to denote in particular a straight chain, branched chain, or cyclic hydrocarbon groups usually having from 1 to 20 carbon atoms, preferably having from 1 to 8 carbon atoms. Examples of alkyl groups include methyl, ethyl, propyl, isopropyl, butyl, isobutyl, sec-butyl, tert-butyl, pentyl, neopentyl, hexyl, cyclopropyl, cyclobutyl, cyclopentyl, and cyclohexyl.

In the present invention, "alkoxy groups" is understood to denote in particular a straight chain, branched chain, or cyclic hydrocarbon groups usually having from 1 to 20 carbon atoms, preferably having from 1 to 8 carbon atoms singularly bonded to oxygen (Alk-O-).

In the present invention, "aryl groups" is understood to denote in particular any functional group or substituent derived from an aromatic ring. In particular, the aryl groups can have 6 to 20 carbon atoms preferably 6 to 12 carbon atoms, in which some or all of the hydrogen atoms of the aryl group may or may not be substituted with other groups, especially alkyl groups, alkoxy groups, thioalkyl groups, aryl groups, hydroxyl groups or halogens such as fluoro. The aryl groups are preferably optionally substituted phenyl groups, naphthyl groups, anthryl group and phenanthryl group.

In the present invention, "aryloxy groups" is understood to denote in particular the aryl group as defined above singularly bonded to oxygen (Ar-O-).

In the present invention, "heterocycles" is understood to denote in particular a cyclic compound which has at least one heteroatom as a member of its one or more rings. Frequent heteroatoms within the ring include sulfur, oxygen and nitrogen. The heterocycles can be either saturated or unsaturated, and may be 3-membered, 4-membered, 5-membered, 6-membered or 7-membered ring. The heterocycles can be further fused with other one or more ring systems. Examples of the heterocycles include pyrrolidines, oxolanes, thiolanes, pyrroles, furans, thiophenes, piperidines, oxanes, thianes, pyridines, quinolines, pyrans, and thiopyrans, and their derivatives. A particular class of the heterocycles includes substituents comprising thiophene moiety. The heterocycles can further be substituted by other groups, such as alkyl groups, alkoxy groups, aryl groups or aryloxy groups as defined above.

In the present invention, "halogenated" is understood to denote in particular at least one of the hydrogen atoms of the respective chemical group has been replaced by a halogen atom, preferably selected from fluorine and chlorine, more preferably fluorine. If all of the hydrogen atoms have been replaced by halogen atoms, the halogenated chemical group is perhalogenated. For instance, "halogenated alkyl groups" include (per)fluorinated alkyl groups such as (per)fluorinated methyl, ethyl, propyl, isopropyl, butyl, isobutyl, *sec*-butyl or *tert*-butyl ; and for instance -CF₃, -C₂F₅, heptafluoroisopropyl (-CF(CF₃)₂), hexafluoroisopropyl (-CH(CF₃)₂) or -CF₂(CF₂)₄CF₃. Non-limiting example of "halogenated aryl groups" is -C₆F₅.

In the present invention, "anchoring groups" is understood to denote in particular groups that will allow attachment of the dyes onto a semiconductor, such as TiO₂ and TiOF₂. Suitable anchoring groups are for instance selected from, but not limited to, the group consisting of -COOH, -PO₃H₂, -PO₄H₂, -PO₂HR⁴ (wherein R⁴ is selected from alkyl groups or aryl groups), -SO₃H, -CONHOH, acetylacetonate, acrylic acid derivatives, malonic acid derivatives, rhodanine-3-acetic acid, propionic acid, deprotonated forms of the aforementioned groups, salts of said deprotonated forms, and chelating groups with π-conducting character. More preferred anchoring groups are -COOH or the salts of deprotonated -COOH. The acrylic acid derivatives may for instance be selected from groups of formula -CH=C(R¹)-COOH where R¹ is selected from H, CN, -COOH and optionally halogenated alkyl groups, preferably from H, CN, -COOH and CF₃. The malonic acid derivatives suitable as anchoring groups may for example be selected from groups of formula -CR³=C(COOH)₂ where R³ is selected from H and optionally halogenated alkyl groups, especially from H and optionally fluorinated alkyl groups. Especially preferable salts of -COOH are for instance ammonium salts, or alkali metal salts, more preferably -COOTBA (wherein, TBA indicates tetrabutylammonium). Further, examples of the anchoring groups include -NO₂ and -P(=O)(Rb)(OH) wherein Rb is selected from the groups consisting of optionally branched C1-C18 alkyl groups and optionally substituted C5-C12 aryl groups, particularly from optionally branched C6-C12 alkyl groups and optionally substituted C6 aryl groups, more particularly from linear C6-C12 alkyl groups and the phenyl group, and halogenated derivatives thereof, such as CF₃.

In the present invention, the anchoring groups can be linked to the azulenocyanine analogue moiety by an optional electronically conjugating bridge. Said optional bridge can be selected from aromatic ring systems, alkene groups, polyene systems, alkyne groups, polyyne systems or heteroatom-containing variants of such systems wherein one or more carbon atoms and/or one or more C=C double bonds are replaced by a heteroatom, preferably from aromatic ring systems and polyene systems.

In one embodiment, the azulenocyanine analogue compound according to the present invention is a condensation product of optionally substituted azulene rings and one another aromatic unit bearing at least one anchoring group, which can form a macrocyclic structure together with the three optionally substituted azulene rings, in a molar ratio of 3:1.

In one another embodiment, the another aromatic unit comprises at least one structure selected from the group consisting of aryl groups, heterocycles and any combination thereof. Preferably, the another aromatic unit comprises benzene ring, biphenyl ring, thiophene ring or thienyl benzene ring structure.

In another embodiment, the azulenocyanine analogue macrocyclic compound according to the present invention corresponds to the formula (I) or its regioisomers : 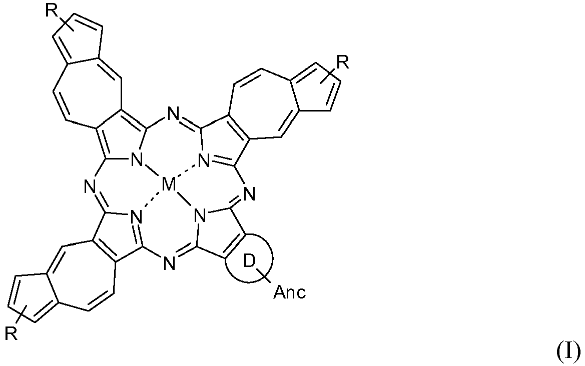 wherein :
M is selected from the group consisting of 2H (metal free analogue), metal cations in the oxidation state +2, preferably Zn(II), Mg(II), Cd(II), Cu(II), Ni(II),
cations in the oxidation state >+2, preferably Ti(IV)O, V(IV)O, Al(III)X, Si(IV)X₂, Sn(IV)X₂, and Ge(IV)X₂, wherein X is selected from halides, OH and ORa with Ra being an alkyl groups or aryl groups ;
R stands for one or more substituents, and is independently selected from the group consisting of hydrogen, hydroxyl, nitro, cyano, halogens, such as fluoro, alkyl groups, halogenated alkyl groups, such as fluorinated alkyl groups, alkoxy groups, halogenated alkoxy groups, such as fluorinated alkoxy groups, aryl group, halogenated aryl groups, such as fluorinated aryl groups, aryloxy groups, halogenated aryloxy groups, such as fluorinated aryloxy groups, -OC-R' (wherein R' is selected from hydrogen, alkyl group, halogenated alkyl groups, such as fluorinated alkyl groups, aryl groups, and halogenated aryl groups, such as fluorinated aryl groups), -CH₂-R" (wherein R" is selected from the group consisting of alkoxy groups, halogenated alkoxy groups, such as fluorinated alkoxy groups, aryoxy groups, and halogenated aryloxy groups, such as fluorinated aryloxy groups, and -NR'₂), -N=N-R', -COH(R')₂, -CH=CH-R', and -C≡C-R';
Anc is one or more anchoring groups ; and
D is selected from optionally substituted aryl groups and heterocycles fused with a pyrrole moiety in the cyclic compound, at least bearing Anc.

In the present invention, the regioisomers of the formula (I) includes the structures below :

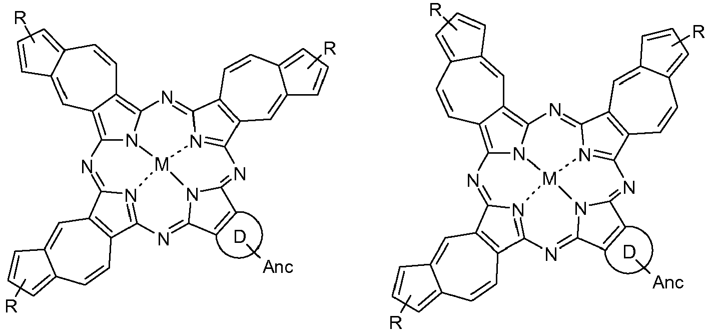

In the formula (I) according to the present invention, R can be one, two or three substituents, each substituted in 1 (or 1', 1"), 2 (or 2', 2"), and/or 3 (or 3', 3") position(s) of the azulene moiety. In one aspect, the one substituent R can be in 1,1',1" position of the azulene moiety. In a preferred aspect, the two substituents R can be in 1,1',1" and 3,3',3" positions.

In a further embodiment, the azulenocyanine analogue compound according to the present invention comprises the anchoring group selected from the group consisting of -COOH, -PO₃H₂, -P(=O)(Rb)(OH) (wherein Rb is selected from the groups consisting of optionally branched C1-C18 alkyl groups and optionally substituted C5-C12 aryl groups, and halogenated derivatives thereof), -PO₄H₂, -PO₂HR⁴, -SO₃H, -CONHOH, -NO₂, acetylacetonate, acrylic acid derivatives, malonic acid derivative, rhodanine-3-acetic acid, propionic acid, deprotonated forms of the aforementioned, salts of said deprotonated forms, and chelating groups with π-conducting character, in particular from the group consisting of -COOH, -PO₃H₂, -P(=O)(Rb)(OH), -SO₃H, and salts of the deprotonated forms of the aforementioned, wherein the anchoring group is linked to D by an optional electronically conjugating bridge.

In a certain embodiment, said optional electronically conjugating bridge is selected from the group consisting of aromatic ring systems, alkene groups, polyene systems, alkyne groups, polyyne systems, heteroatom-containing variants of such groups or systems wherein one or more carbon atoms and/or one or more C=C double bonds are replaced by a heteroatom, and any combination thereof, in particular from benzene ring systems, alkyne groups or any combination thereof.

In a certain other embodiment, the azulenocyanine analogue compound according to the present invention has any one of the formulae (II), (III) or (IV) below : 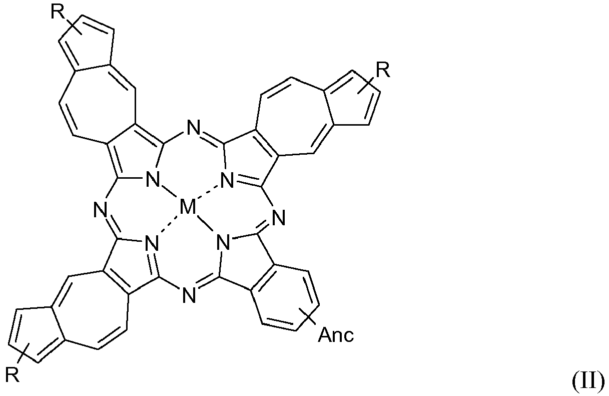 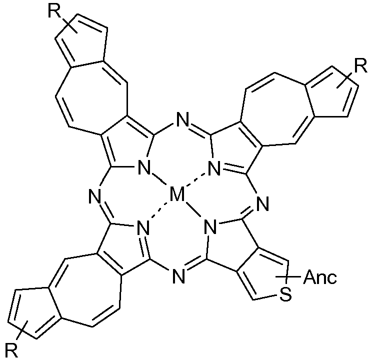 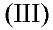 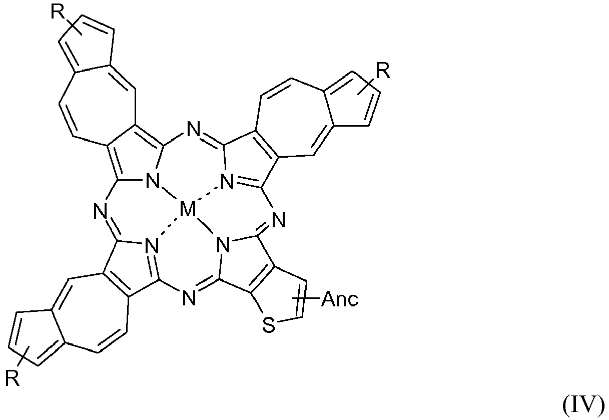 wherein M, R and Anc have the same meaning as denoted in the above.

In a specific embodiment, the azulenocyanine analogue compound according to the present invention is selected from the structures below : 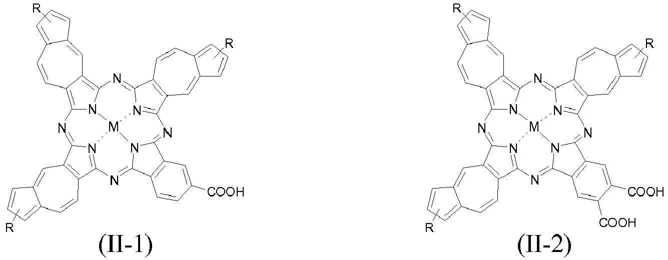 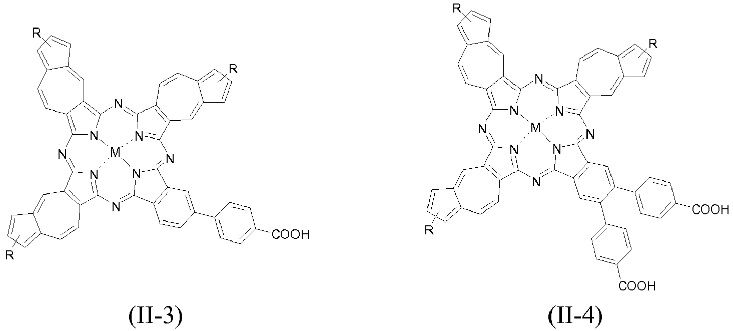 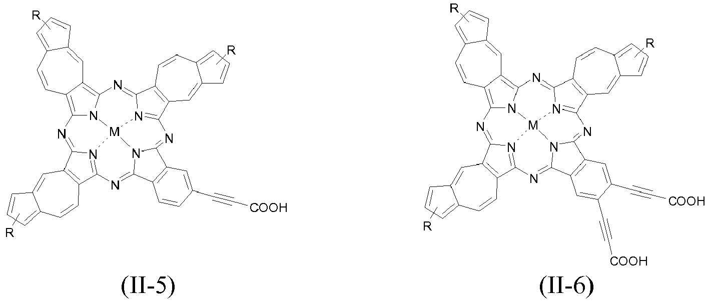 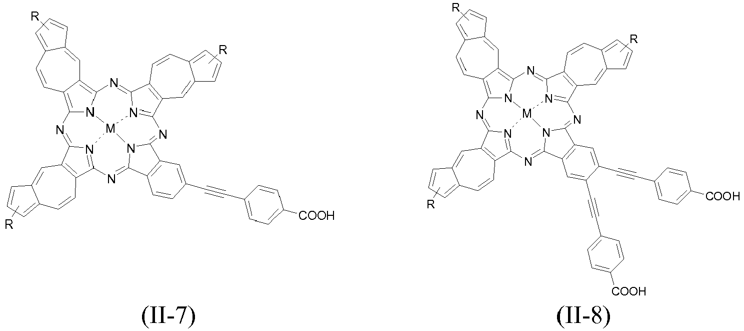 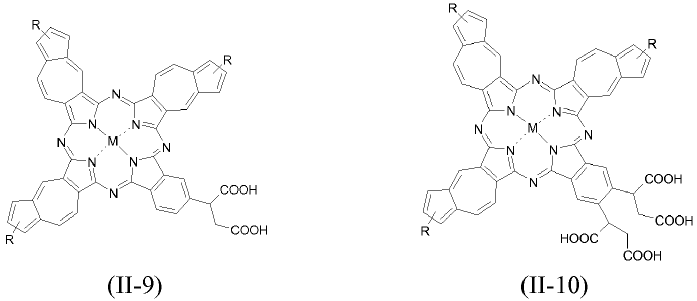 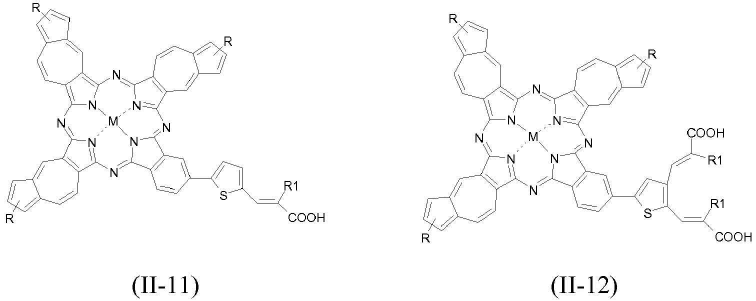 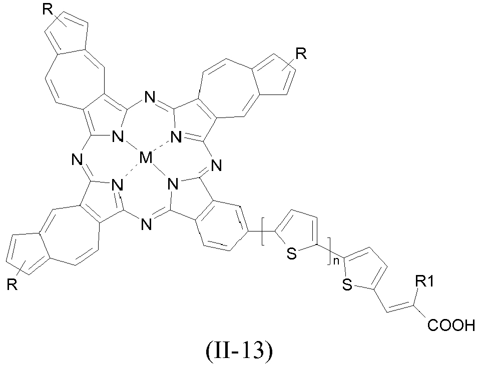 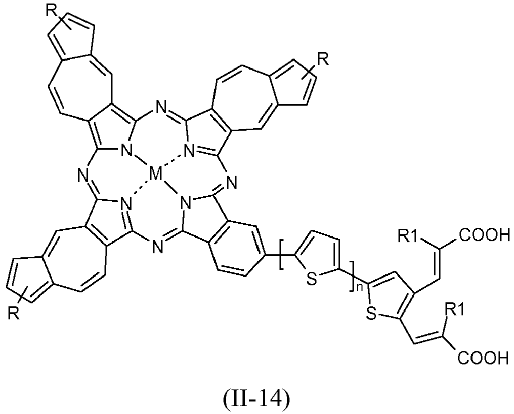 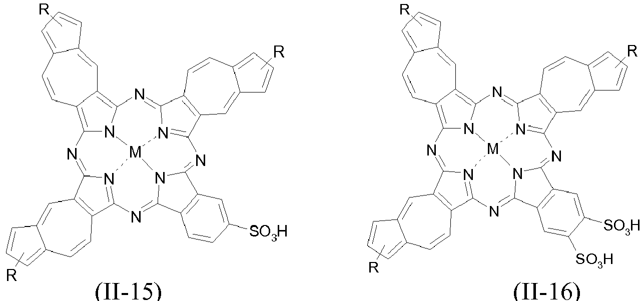 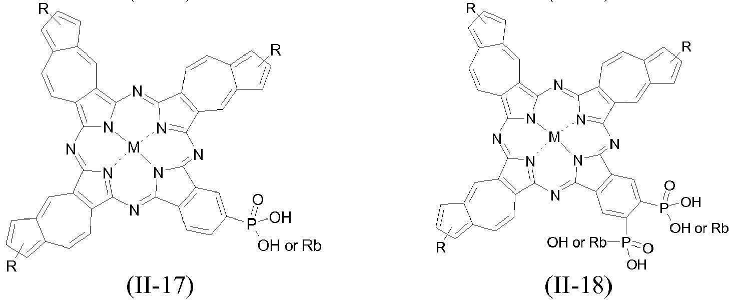 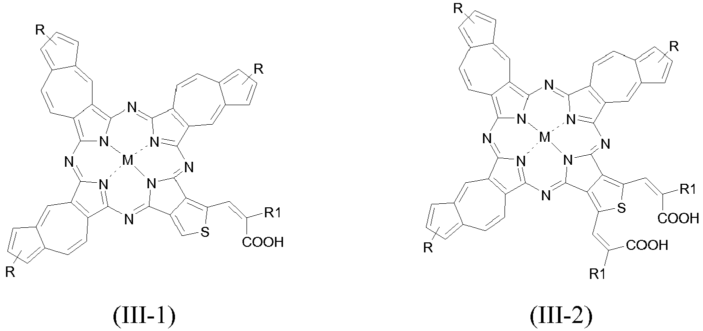 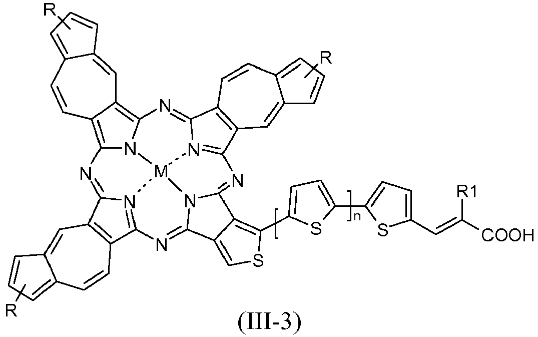 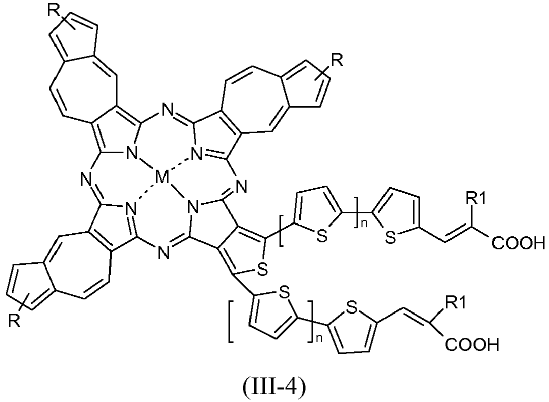 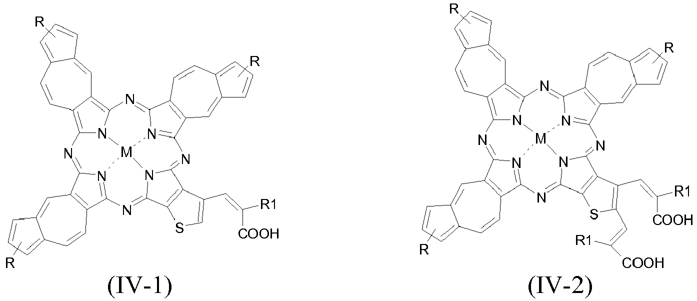 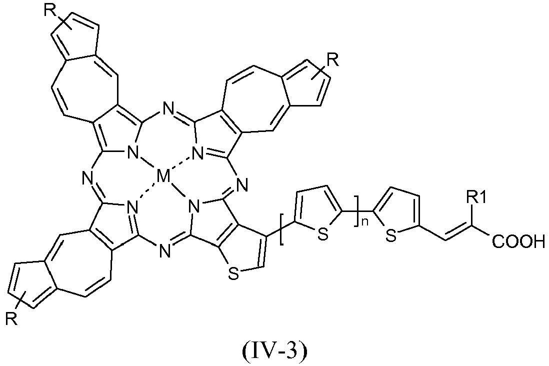 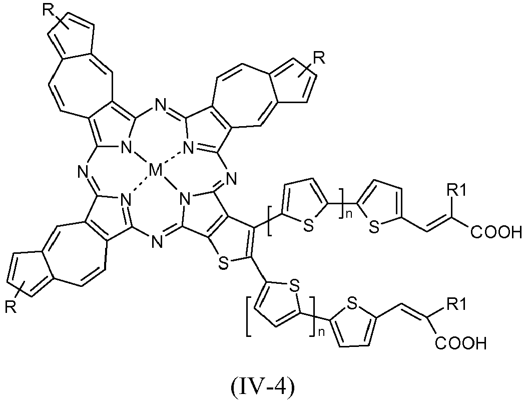 wherein M, R and Rb have the same meaning as denoted in the above, R1 is -CN, -COOH or optionally halogenated alkyl groups, and n is 0, 1, 2, 3, 4 or 5.

In a further specific embodiment, the azulenocyanine analogue compound is a fluorinated compound. For instance, the azulene ring moiety can be fluorinated, preferably R can be fluorine or fluorinated substituent. Also, said another aromatic unit moiety can be fluorinated, preferably through R1 and/or Rb. Further, the fluorination can occur both in the azulene ring moiety and the another aromatic unit moiety.

The azulenocyanine analogue compounds according to the present invention described herein are suitable for use in photoelectric conversion devices, particularly in dye-sensitized solar cells (DSSC) or in solid-state organic photovoltaics (OPV).

Therefore, the present invention also relates to use of the azulenocyanine analogue compounds according to the present invention as a dye suitable for use in dye-sensitized solar cells (DSSC). Further, the present invention concerns use of the azulenocyanine analogue compounds according to the present invention as a dye suitable for use in solid-state organic photovoltaic (OPV).

Still another embodiment of the present invention concerns a method of production of photoelectric conversion devices, in particular dye-sensitized solar cells (DSSC) or in solid-state organic photovoltaics (OPV), wherein the azulenocyanine analogue compound according to the present invention is used.

Especially, the DSSC offers the prospect of a cheap and versatile technology for large scale production of solar cells. The dye-sensitized solar cell (DSSC) is formed by a combination of organic and inorganic components that could be produced at a low cost. The dye-sensitized solar cells have advantages over silicon-based solar cells in terms of simplified processing steps, low fabrication cost, transparency and pleochroism. The dye-sensitized solar cells can be fabricated from flexible substrates to function as cells of mobility and portability. Dye-sensitized solar cells have also the advantage to be lightweight.

The dye-sensitized solar cells have lower energy (photoelectric) conversion efficiency over that of the silicon-based solar cells such that a wide range of researches are briskly under way to enhance the energy conversion efficiency. In order to improve the energy conversion efficiency, extension of wave length up to infrared regions is being waged with great concern. The energy bandgap (eV) for use in solar cells must exceed 1.4 eV (electron volt).

The basic element of a DSSC is generally a TiO₂ (titanium dioxide) nanoparticulate structure sensitized with dye molecules to form the core of a DSSC. The assembly of titanium dioxide nanoparticles is well connected to their neighbors. TiO₂ is the preferred material for the nanoparticles since its surface is highly resistant to the continued electron transfer. However, TiO₂ only absorbs a small fraction of the solar photons (those in the UV). The dye molecules attached to the semiconductor surface are used to harvest a great portion of the solar light.

In this context, the azulenocyanine analogue compound of the present invention can be used as a dye, in particular as a sensitizing dye, in such device or cell. Accordingly, the present invention further relates to a photoelectric conversion device, preferably a dye-sensitized solar cell (DSSC), which comprises the azulenocyanine analogue compound of the present invention.

In a preferred embodiment, the present invention relates to a dye-sensitized solar cell comprising at least one fluorinated azulenocyanine analogue compound according to the present invention as a dye and at least TiOF₂ as semiconductor.

The fluorinated azulenocyanine analogue compounds according to the present invention are especially advantageous, in particular when combined with TiOF₂ used as semiconductor in dye sensitized solar cells. Indeed, without being bound by any theory, it is believed that the conduction band edge of TiOF₂ is lower in energy compared to the conduction band of TiO₂ while the fluorinated azulenocyanine analogue compounds of the present invention exhibit a lower LUMO level compared to similar non-fluorinated compounds. Such combination can be thus especially advantageous.

The present invention further relates to a method for making the above-mentioned azulenocyanine analogue compounds. The method comprises a step of reacting at least one azulene dinitriles and at least one other aromatic dinitriles different from the azulene dinitriles as building blocks for the macrocyclic structure.

The examples of the azulene dinitrile for said method include the following structures : 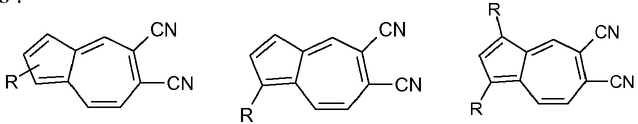 wherein R has the same meaning as denoted above.

In the present invention, said azulene dinitriles can be synthesized by electrophilic aromatic substitution by the electrophile E⁺ in the 1- or 1,3-positions of the azulene ring, as exemplified below :

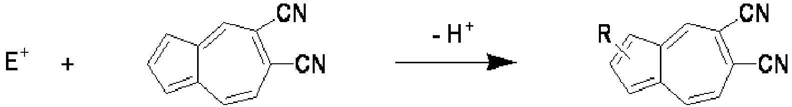

The mechanism for electrophilic aromatic substitution is selected from Friedel-Crafts-alkylation, Friedel-Crafts-acylation, chloromethylation, diazo coupling reaction, hydroxyalkylation, or Suzuki coupling / Heck coupling / Stille coupling reaction.

In case of Friedel-Crafts-alkylation, said R-substituted azulene dinitriles can be prepared by reaction with R-X, with a preference for R being alkyl groups, aryl groups, fluorinated alkyl groups or fluorinated aryl groups.

In case of Friedel-Crafts-acylation, organic acid chlorides or acid anhydrides can be used to form -OC-R' substituted azulene dinitriles, with a preference for R' being alkoxy groups, aryloxy groups, fluorinated alkoxy groups or fluorinated aryloxy groups.

In case of chloromethylation, Cl-CH₂ substituted azulene dinitrile is first prepared. Then, as one way, subsequent reactions with non-fluorinated and fluorinated alcohols, phenols and heterocycles to form -CH₂-R" substituted azulene dinitriles, with a preference for R" being alkoxy groups, aryloxy groups, fluorinated alkoxy groups or fluorinated aryloxy groups. The another way is to subsequently react the -Cl-CH₂ substituted azulene dinitrile with non-fluorinated and fluorinated aliphatic and aromatic/heterocyclic amines to for -CH₂-R" substituted azulene dinitriles, with a preference for R" being -NR'₂ (wherein R' is alkyl groups, aryl groups, fluorinated alkyl groups or fluorinated aryl groups).

In case of diazo coupling, reaction with diazonium salts can be performed to result in -N=N-R', with a preference for R' being aryl groups or fluorinated aryl groups.

In case of hydroxyalkylation, -COH(R')₂ substituted azulene dinitriles, with a preference for R' being alkyl groups, aryl groups, fluorinated alkyl groups or fluorinated aryl groups.

In case of Suzuki coupling / Heck coupling / Stille coupling reaction, first 5,6-dicyanoazulene halide can be first converted to R' substituted azulene dinitrile, and then converted to -CH=CH-R' substituted azulene dinitrile, with a preference for R' being alkyl groups, aryl groups, fluorinated alkyl groups or fluorinated aryl groups.

In case of ethynyl bonds, iodoazulenes obtained from 5,6-dicyanoazulene with *N*-iodosuccinimide are reacted with an alkylacetylene, arylacetylene or trimethylsilylacetylene in the presence of a Pd catalyst and CuI to obtain 5,6-dicyanoazulenes with R-≡-R"" (wherein R"" is selected from alkyl groups, aryl groups and trimethylsilyl group). The trimethylsilyl group can be splitted to obtain R-C≡-H.

In a particular embodiment, the method for making the azulenocyanine analogue compounds according to the present invention further comprises a step of utilizing an M-containing precursor. The M-containing precursor is preferably a metal salt or derivative of M, more preferably a Zn salt such as zinc acetate, magnesium oxide, an aluminum derivative such as aluminum chloride or bromide, a silicon derivative such as tetrachlorosilane, a stannous halide (SnHal₂), or a tin derivative such as tin dichloride (SnCl₂).

In the present invention, the at least one other aromatic dinitriles different from the azulene dinitriles as building block used in the method for making the azulenocyanine analogue compounds are preferably the dinitriles having aromatic structure. The examples for such aromatic structure include phthalonitriles, phthalonitriles substituted by phenyl group(s), dicyanothiophenes, and dicyanothiophenes substituted by phenyl group(s). More preferably, the at least one other aromatic dinitrile can be selected from the group consisting of : 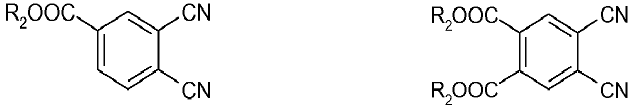 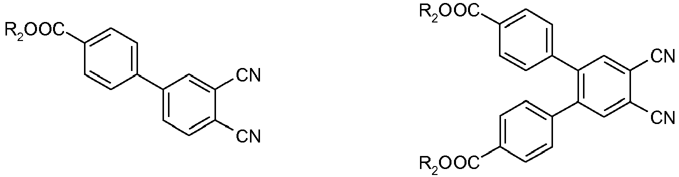 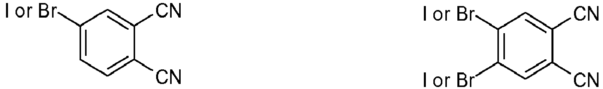 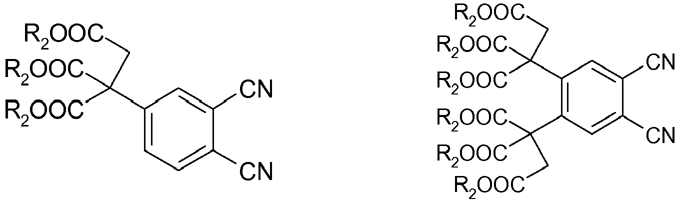 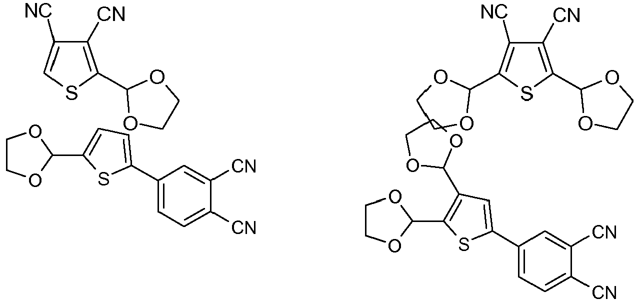 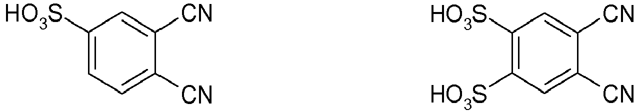 wherein R2 is selected from alkyl groups, preferably methyl or ethyl group.

According to a first embodiment, it is possible to react the azulene dinitriles and the other aromatic dinitriles than the azulene dinitriles as building blocks for the macrocyaclic structure in the optional presence of a M-containing precursor, preferably a metal salt or derivative of M to form the azulenocyanine analogue compound of the present invention by statistical cyclotetramerization. Such reaction is typically performed by reacting the azulene dinitrile with the other aromatic dinitriles different from the azulene dinitrile in a molar ratio about 3:1. The exemplary reaction schemes are as follows :

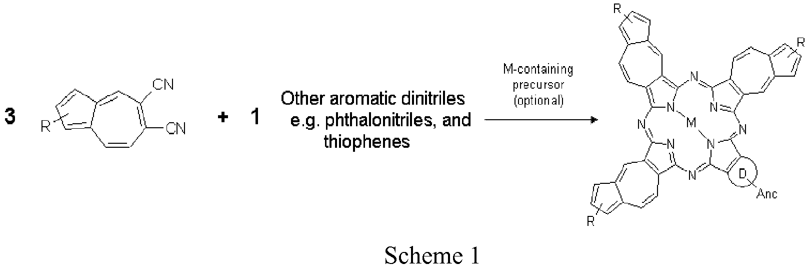

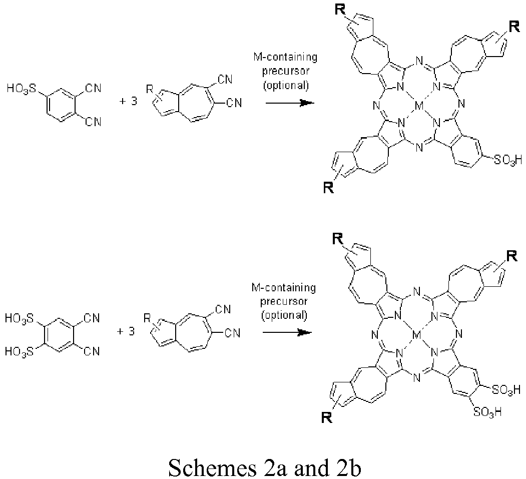

According to a second embodiment, it is possible to first react the azulene dinitriles and the other aromatic dinitriles different from the azulene dinitriles as building blocks for the macrocyclic structure in the optional presence of an M-containing precursor, preferably a metal salt or derivative of M to form an azulenocyanine analogue compound by statistical cyclotetramerization, and to further react the resulting compound to form the azulenocyanine analogue compound of the present invention. In such second embodiment, the reaction is typically performed by reacting the azulene dinitrile with the other aromatic dinitrile in a molar ratio about 3:1, in the optional presence of a M-containing precursor, preferably a metal salt or derivative of M. The exemplary reaction schemes for this embodiment are as follows :

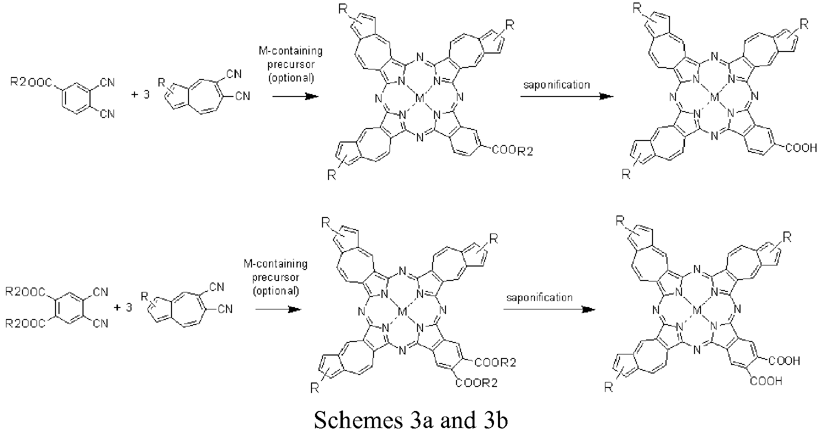

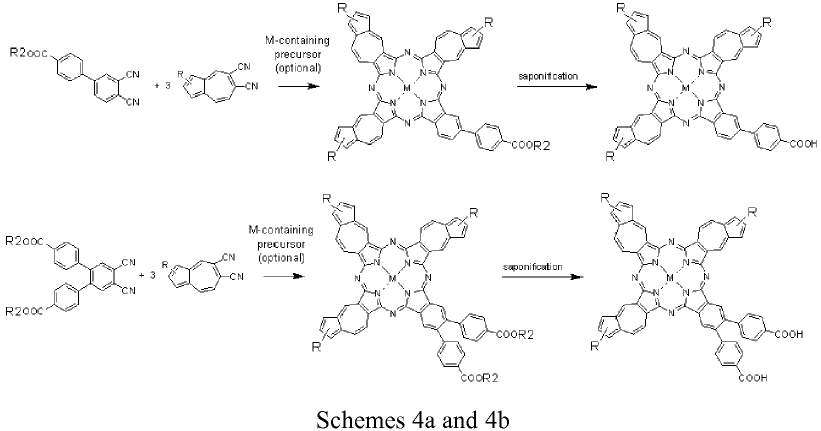

In the schemes 3a, 3b, 4a and 4b, alkyl esters (with the alkyl being for instance methyl, ethyl, propyl, butyl or pentyl) of phthalonitriles are employed in the reaction with the azulene dinitrile derivatives. Then in the formed azulenocyanine analogue esters, the ester groups are hydrolyzed to obtain the azulenocyanine analogue compounds with free carboxylic acid groups.

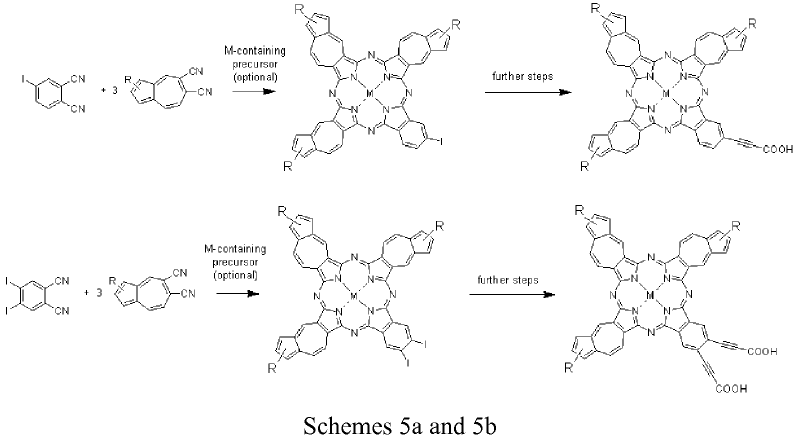

In the schemes 5a and 5b, the azulenocyanine analogue compounds with one or two ethynyl carboxylic acid groups are obtained as follows. Firstly, from iodophthalonitriles and azulene dinitrile derivatives, the azulenocyanine analogue compounds with iodo groups are synthesized. Secondly, the compounds are converted with propargyl alcohol in the presence of a Pd catalyst to azulenocyanine analogue compounds with -CH₂OH groups. And then, the -CH₂OH groups are reacted with 2-iodoxybenzoic acid to obtain the azulenocyanine analogue compounds with -COH groups. Finally, the -COH groups are oxidized with e.g. NaClO₂ to obtain the azulenocyanine analogue compounds with ethynyl carboxylic acid groups.

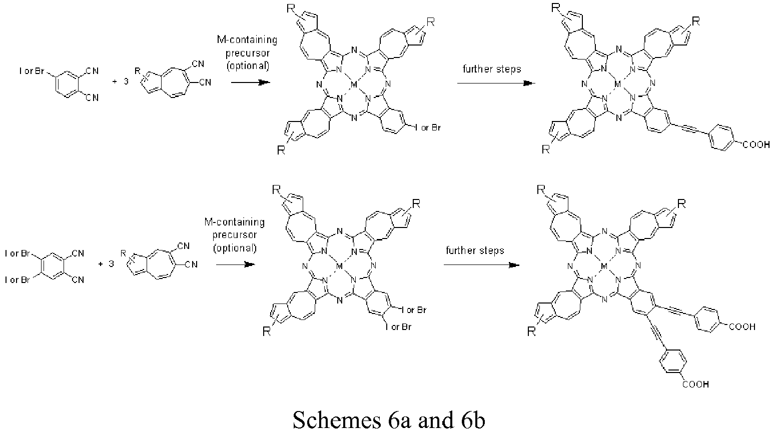

In the schemes 6a and 6b, the azulenocyanine analogue compounds with one or two ethynyl phenylene carboxylic acid groups are obtained as follows. Firstly, as described in the fist step of the schemes 5a and 5b. Secondly, the reaction with trisisopropylacetylene is carried out to obtain azulenocyanine analogue compounds with triisopropylsilyl-ethynyl groups. Finally, the triisopropylsilyl-ethynyl groups are reacted in the presence of a Pd catalyst to obtain the azulenocyanine analogue compounds with ethynyl phenylene carboxylic acid groups.

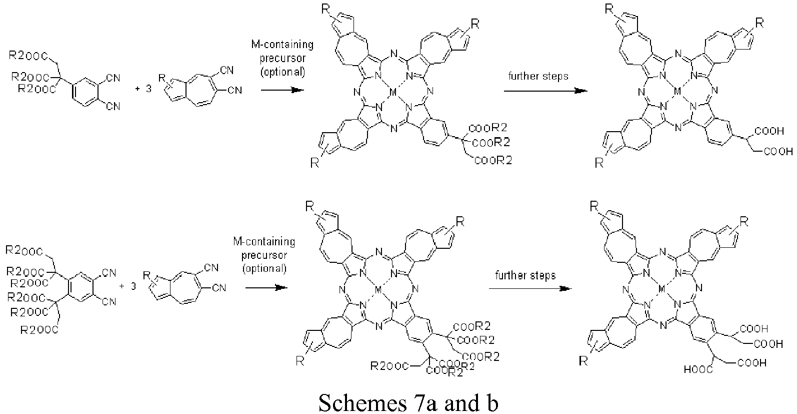

In the schemes 7a and 7b, the azulenocyanine analogue compounds with succinyl groups are obtained as follows. Fristly, the azulenocyanine analogue compounds with, for example, tricarbethoxyethyl groups are synthesized from phthalonitrile derivatives with tricarbethoxyethyl groups and azulene dinitrile derivatives. Then, by saponification, the tricarbethoxyethyl groups are converted to obtain the azulenocyanine analogue compounds with free succinyl groups.

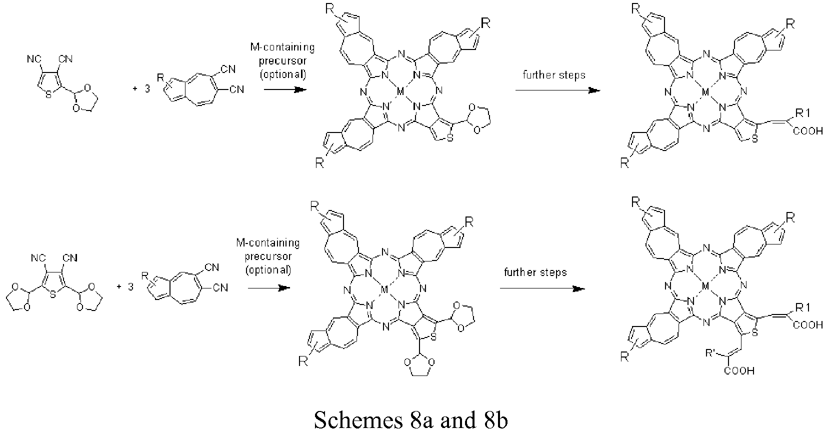

In the schemes 8a and 8b, the azulenocyanine analogue compounds with thieno acrylic acid groups are obtained as follows. Firstly, exemplarily, 3,4-dicyanothiophene with by ethylene glycol protected aldehyde groups are reacted with azulene dinitrile derivatives to obtain azulenocyanine analogue compounds with protected thieno aldehyde groups. Secondly, the protected aldehyde groups are deprotected to obtain azulenocyanine analogue compounds with free aldehyde groups. Finally, for example, the azulenocyanine analogue compounds with free aldehyde groups are reacted with cyano acetic acid to obtain azulenocyanine analogue compounds with, for example, cyano acrylic acid groups.

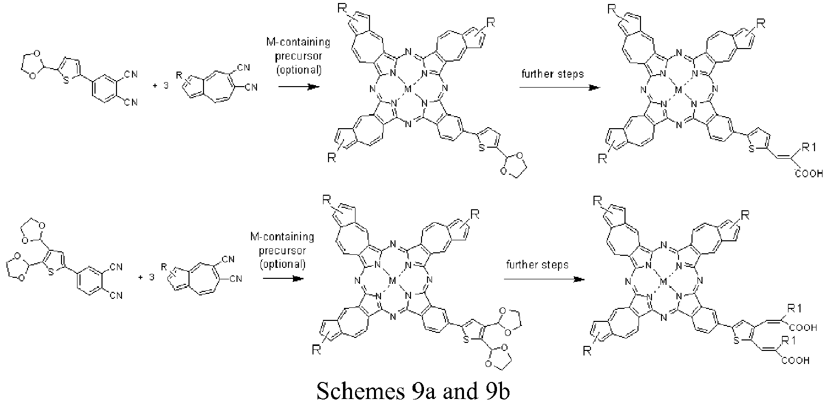

In the schemes 9a and 9b, the reaction sequence is analogous to the schemes 8a and 8b. For example, in the first step, thiophene derivatives substituted by phthalonitrile and protected aldehyde groups are employed in the reaction with azulene dinitrile derivatives. Finally, azulenocyanine analogue compounds with thienyl substituted by cyano acrylic acids are obtained.

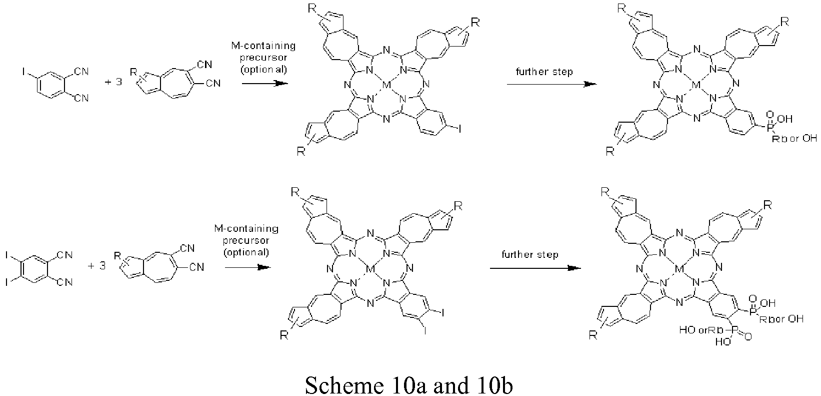

In the schemes 10a and 10b, the first step is analogous as described for the schemes 5a and 5b. And then, the azulenocyanine analogue compounds with iodo groups are converted with ethyl alkyl-H-phosphinate (with the alkyl being, for example, octyl) to obtain azulenocyanine analogue compounds with ethyl alkyl-phosphinate groups. Finally, by saponification, the azulenocyanine analogue compounds with free phosphinic acid groups are obtained.

The macrocyclic compounds according to the present invention may be further isolated, for instance by column chromatography, preferably by high pressure liquid chromatography (HPLC).

While preferred embodiments of this invention have been shown and described, modifications thereof can be made by one skilled in the art without departing from the spirit or teaching of this invention. The embodiments described herein are exemplary only and are not limiting. Many variations and modifications of systems and methods are possible and are within the scope of the invention. Accordingly, the scope of protection is not limited to the embodiments described herein, but is only limited by the claims that follow, the scope of which shall include all equivalents of the subject matter of the claims.

Should the disclosure of any patents, patent applications, and publications which are incorporated herein by reference conflict with the description of the present application to the extent that it may render a term unclear, the present description shall take precedence.

### Brief Description of the Drawing

Figure 1 shows the electronic absorption spectrum of an azulenocyanine analogue compound (mixture of regioisomers).

Figure 2 shows the calculation of the main NIR transitions of the regioisomers of formula (I) in relation to the conduction band level of TiO₂ and the redox couple 3I⁻/I₃⁻.

### Examples

### Example 1 : Preparation of azulenocyanine analogue compounds as mixture of regioisomers with two carboxylic acid groups according to Scheme 3b as mixture of regioisomers (Scheme 11)

### Step 1 : Synthesis of the diesters of the azulenocyanine analogue compounds.

3 mmol of unsubstituted 5,6-dicyanoazulene or 1,3-disubstituted 5,6-dicyanoazulenes (wherein substituted by R in 1- and 3-position, the R = -F, -C(CH₃)₃, -CO-CF₃, -O-CH₂CF₃, or (2,6-diphenyl)phenoxy), 1 mmol of 4,5-dipentoxycarbonylphthalonitrile and 1.4 mmol of dry Zn(OAc)₂ are suspened in 50 mL dry 1-pentanol. The mixture is heated under inert gas and stirring at 145°C. 2 mmol of DBU is added, and heating is continued for 24 h at 145°C. The cooled reaction mixture is added to methanol, and the precipitate is filtered. Then the azulenocyanine analogue compounds are dissolved in CHCl₃ under addition of a small amount of DMF (in dependence of the azulene derivative) and isolated by column chromatography on silica gel using CHCl₃ as solvent (with addition of a small amount of DMF). The black coloured azulenocyanine analogue diesters are obtained in dependence of the kind of employed azulene derivatives in yields of 8 till 25 %.

### Step 2 : Synthesis of azulenocyanine analogue compounds with two carboxylic acid groups.

To a suspension of 0.1 mmol of diesters in 20 mL 1-pentanol is added 40 mmol powdered KOH. The mixture is stirred for 5 days at room temperature. Then, 10 % HCl is added to neutralize the unreacted KOH, and the pH is adjusted to 2. The azulenocyanine analogue compounds are isolated by filtration, washed with slightly acidic water and dried. The black coloured azulenocyanine analogue compounds with two carboxylic acid groups are obtained in dependence of the kind of employed azulene derivatives in yields of 50 till 75 %.

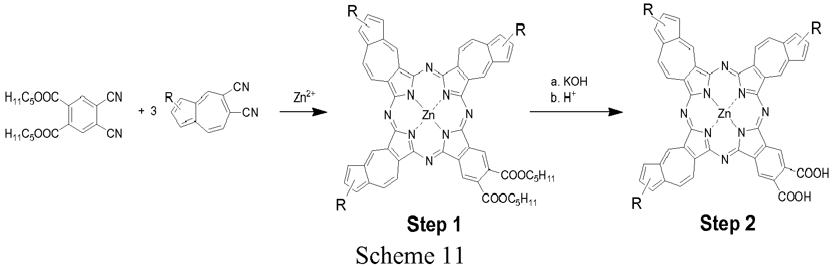

### Example 2 : Preparation of azulenocyanine analogue compounds as mixture of regioisomers with a phenylene carboxylic acid group according to Scheme 4a as mixture of regioisomers (Scheme 12)

### Step 1 : Synthesis of azulenocyanine analogue compounds with a phenylene carboxylic acid ester group.

A mixture of 3 mmol of unsubstituted 5,6-dicyanoazulene or 1,3-disubstituted 5,6-dicyanoazulenes (wherein substituted by R in 1- and 3-position, the R = -F, -C(CH₃)₃, -CO-CF₃, -O-CH₂CF₃, or (2,6-diphenyl)phenoxy), 1 mmol of 4-methoxycarbonylphthalonitrile and 2 mmol of dry ZnCl₂ are suspened in 50 mL dry dimethylaminoethanol. The mixture is heated under inert gas and stirring at 145 °C. 2 mmol of DBU is added, and heating is continued for 72 h at 145 °C. The solvent is removed in vacuo, and the residue is washed with methanol. Then, the azulenocyanine analogue compounds are dissolved in CHCl₃ under addition of a small amount of DMF (in dependence of the azulene derivative) and isolated by column chromatography on silica gel or activated alumina using CHCl₃ as solvent (with addition of a small amount of DMF). The black coloured azulenocyanine analogue esters are obtained in dependence of the kind of employed azulene derivatives in yields of 5 till 26 %.

Step 2 : Synthesis of azulenocyanine analogue compounds with a phenylene carboxylic acid group. To 1 mmol of diesters are added 20 mL 1.0 M aqueous NaOH in 75 mL THF. The mixture is stirred for 2 days at 70°C. The solvents are removed in vacuo. The residue is dissolved in 100 mL water, refluxed for 1 h, filtered and neutralized with acetic acid. The resulting precipitate is filtered and dried. The black coloured azulenocyanine analogue compounds with a phenylene carboxylic acid group are obtained in dependence of the kind of employed azulene derivatives in yields of 65 till 80 %.

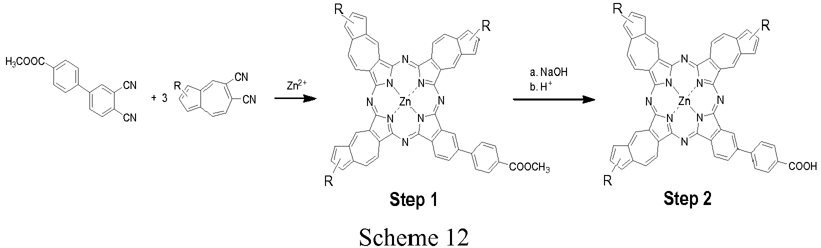

### Example 3 : Preparation of azulenocyanine analogue compounds as mixture of regioisomers with a succinyl group according to Scheme 7a as mixture of regioisomers (Scheme 13)

### Step 1 : Synthesis of azulenocyanine analogue compounds with a tricarbethoxyethyl group.

A mixture of 3 mmol of unsubstituted 5,6-dicyanoazulene or 1,3-disubstituted 5,6-dicyanoazulenes (wherein substituted by R in 1- and 3-position, the R = -F, -C(CH₃)₃, -CO-CF₃, -O-CH₂CF₃, or (2,6-diphenyl)phenoxy), 1 mmol of 4-(1,1,2-tricarbethoxyethyl)phthalonitrile and 2 mmol of dry ZnCl₂ are suspened in 20 mL dry dimethylaminoethanol. 2 mmol of DBU is added, and the mixture is heated under reflux for 48 h. The solvent is removed in vacuo, and the compounds are isolated by silica gel chromatography with CHCl₃ as eluent (under addition of a small amount of DMF in dependence of the azulene derivative) and isolated by column chromatography on silica gel or activated alumina using CHCl₃ as solvent (with addition of a small amount of DMF). The black coloured azulenocyanine analogue compounds with a tricarbethoxyethyl groups are obtained in dependence of the kind of employed azulene derivatives in yields of 8 till 27 %.

### Step 2 : Synthesis of azulenocyanine analogue compounds with a succinyl group.

1 mmol of the azulenocyanine analogue compounds with a tricarbethoxyethyl group are dissolved in 100 mL of ethanol and 2 g of Na is added. The reaction mixture is stirred at room temperature for 7 d and the solvent is then evaporated under reduced pressure. The obtained solid material is re-dissolved in ethanol and the pH value is adjusted to 3 by addition of dilute HCl. The precipitate is filtered and dried. The black coloured azulenocyanine analogue compounds with phenylene carboxylic acid groups are obtained in dependence of the kind of employed azulene derivatives in yields of 75 till 85 %.

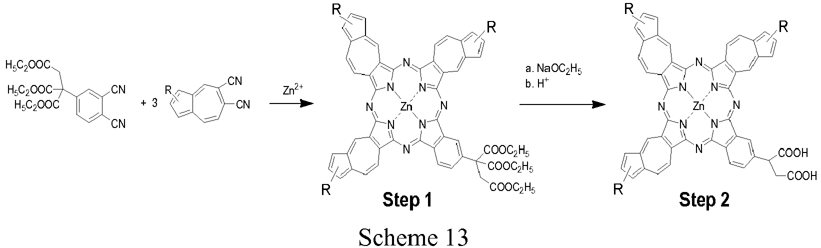

### Example 4 : Preparation of azulenocyanine analogue compounds as mixture of regioisomers with a phosphinic acid group according to Scheme 10a as mixture of regioisomers (Scheme 14)

### Step 1 : Synthesis of azulenocyanine analogue compounds with an iodo group.

To a stirred solution of 3 mL dry 1-hexanol under inert gas 4 mmol of Li shot is added, and the reaction mixture is allowed to warm up to 100°C and stirred. After the Li metal is consumed, 0.51 mmol of 5,6-dicyanoazulene or 1,3-disubstituted 5,6-dicyanoazulenes (wherein substituted by R in 1- and 3-position, the R = -F, -C(CH₃)₃, -CO-CF₃, -O-CH₂CF₃, or (2,6-diphenyl)phenoxy) and 0.17 mmol of 4-iodophthalonitrile are added, and the reaction mixture is warmed up to reflux for 8 h. Then, the reaction mixture is cooled and poured into MeOH containing a few drops of aqueous 2N HCl and filtrated. The isolated product is dissolved in CHCl₃ and the metal-free azulenocyanine analogue compounds with an iodo group are separated by silica gel column chromatography using CHCl₃ as eluent. The metal free compounds are then metalated with Zn(OAc)₂ in DMF. The black coloured azulenocyanine analogue compounds with an iodo group are obtained in dependence of the kind of employed azulene derivatives in yields of 9 till 31 %.

### Step 2 : Synthesis of azulenocyanine analogue compounds with a phosphinic acid group.

To a solution of 0.1 mmol of the azulenocyanine analogue compounds with an iodo group, 2.5 mmol of CsCO₃ and 0.5 mmol of proline in 25 mL dry toluene under inert gas, 12.5 mmol of ethyl octyl-H-phosphinate are added. The mixture is stirred for 30 min at room temperature. Then, 0.13 equivalents of CuI are added and the reaction mixture is heated at 110°C overnight. After cooling, water is added and the mixture is extracted with ethyl acetate. The organic layer is dried over anhydrous Na₂SO₄, and after filtration of the drying agent, the solvents are distilled of. The residue is dissolved in 5 mL CHCl₃ under inert gas, 1 mL triethylamine is added and the mixture is cooled to 0°C. Then, 1 mL trimethylsilyliodide (TMS-I) is added dropwise, and the mixture is heated at 40°C for 2 h. After addition of 20 mL methanol, the mixture is further stirred for 2 h. Water is added, and the mixture is extracted with CHCl₃. The organic layers are washed with 1N HCl and water. After drying of the organic layers with Na₂SO₄, the organic solvents are evaporated. The black coloured azulenocyanine analogue compounds with a phosphinic acid groups are obtained in dependence of the kind of employed azulene derivatives in yields of 60 till 71 %.

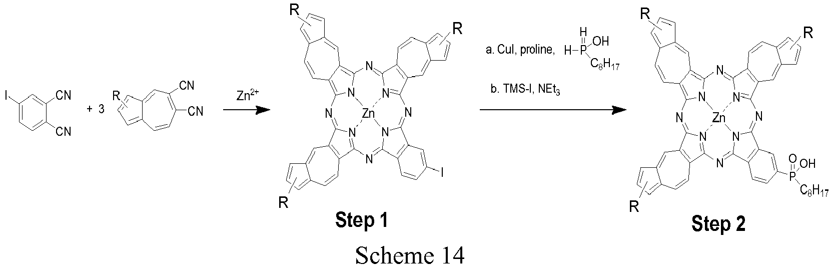

## Claims

1. An azulenocyanine analogue compound comprising at least one anchoring group.

2. The azulenocyanine analogue compound according to Claim 1, which is a condensation product of optionally substituted azulene rings and another aromatic unit bearing at least one anchoring group in a molar ratio about 3:1.

3. The azulenocyanine analogue compound according to Claim 1 or 2, wherein the another aromatic unit comprises at least one structure selected from the group consisting of aryl groups, heterocycles and any combination thereof, preferably benzene ring, biphenyl ring, thiophene ring and thienyl benzene ring.

4. The azulenocyanine analogue compound according to any one of Claims 1 to 3, having the following formula (I) or its regioisomers : 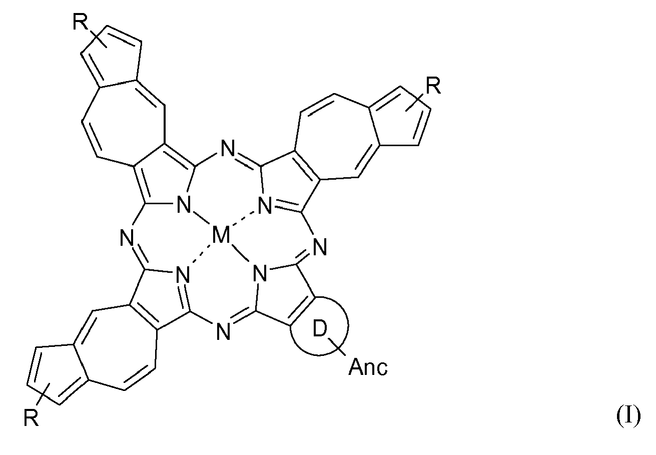 wherein :
M is selected from the group consisting of 2H (metal free analogue), metal cations in the oxidation state +2, preferably Zn(II), Mg(II), Cd(II), Cu(II), Ni(II),
cations in the oxidation state >+2, preferably Ti(IV)O, V(IV)O, Al(III)X, Si(IV)X₂, Sn(IV)X₂, and Ge(IV)X₂, wherein X is selected from halides, OH and ORa with Ra being an alkyl groups or aryl groups ;
R is one or more substituents, and is independently selected from the group consisting of hydrogen, hydroxyl, nitro, cyano, halogens, such as fluoro, alkyl groups, halogenated alkyl groups, such as fluorinated alkyl groups, alkoxy groups, halogenated alkoxy groups, such as fluorinated alkoxy groups, aryl group, halogenated aryl groups, such as fluorinated aryl groups, aryloxy groups,
halogenated aryloxy groups, such as fluorinated aryloxy groups, -OC-R' (wherein R' is selected from hydrogen, alkyl group, halogenated alkyl groups,
such as fluorinated alkyl groups, aryl groups, and halogenated aryl groups, such as fluorinated aryl groups), -CH₂-R" (wherein R" is selected from the group consisting of alkoxy groups, halogenated alkoxy groups, such as fluorinated alkoxy groups, aryoxy groups, and halogenated aryloxy groups, such as fluorinated aryloxy groups, and -NR'₂), -N=N-R', -COH(R')₂, -CH=CH-R',
and -C≡C-R';
Anc is one or more anchoring groups ; and
D is selected from optionally substituted aryl groups and heterocycles fused with a pyrrole moiety in the cyclic compound, at least bearing Anc.

5. The azulenocyanine analogue compound according to any one of Claims 1 to 4, wherein Anc is selected from the group consisting of -COOH, -PO₃H₂, -P(=O)(Rb)(OH) (wherein Rb is selected from the groups consisting of optionally branched C1-C18 alkyl groups and optionally substituted C5-C12 aryl groups, and halogenated derivatives thereof), -PO₄H₂, -PO₂HR⁴ (wherein R⁴ is selected from alkyl groups or aryl groups), -SO₃H, -CONHOH, -NO₂, acetylacetonate, acrylic acid derivatives, malonic acid derivative, rhodanine-3-acetic acid, propionic acid, deprotonated forms of the aforementioned, salts of said deprotonated forms, and chelating groups with π-conducting character, in particular from the group consisting of -COOH, -PO₃H₂, -P(=O)(Rb)(OH), -SO₃H, and salts of the deprotonated forms of the aforementioned, and is linked to D by an optional electronically conjugating bridge.

6. The azulenocyanine analogue compound according to any one of Claims 1 to 5, wherein said optional electronically conjugating bridge is selected from the group consisting of aromatic ring systems, alkene groups, polyene systems, alkyne groups, polyyne systems, heteroatom-containing variants of such groups or systems wherein one or more carbon atoms and/or one or more C=C double bonds are replaced by a heteroatom, and any combination thereof, in particular from benzene ring systems, alkyne groups or combination thereof.

7. The azulenocyanine analogue compound according to any one of Claims 1 to 6, having any one of the formulae (II), (III) or (IV) below : 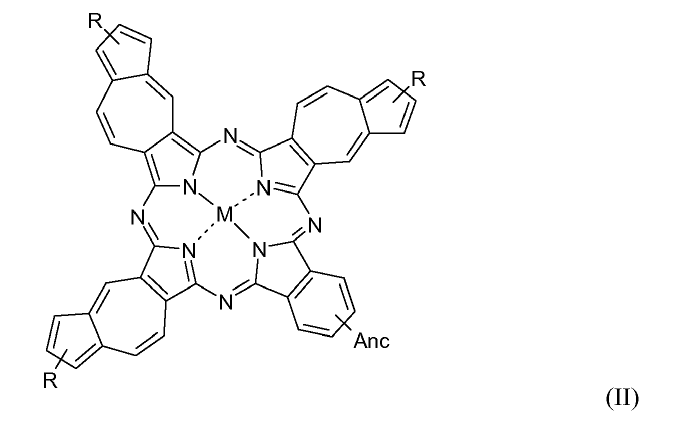 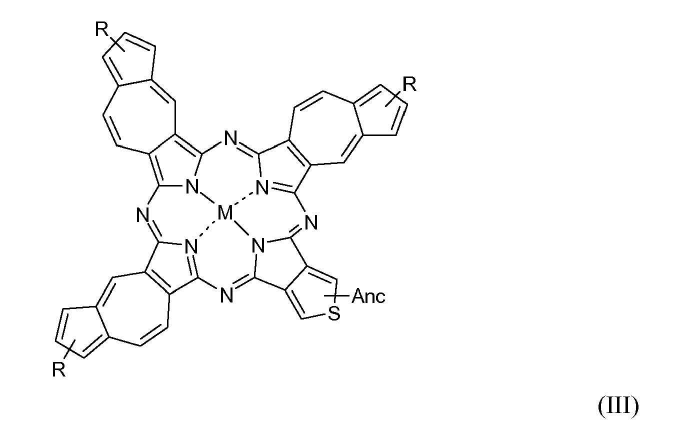 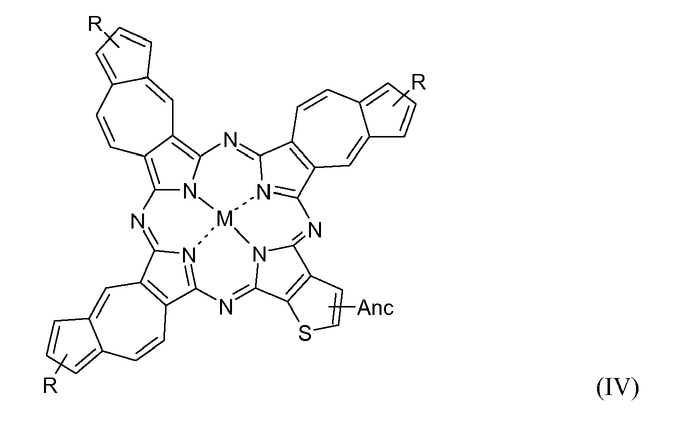 wherein M, R and Anc have the same meaning as denoted in the above.

8. The azulenocyanine analogue compound according to any one of Claims 1 to 7, which is selected from the structures below : 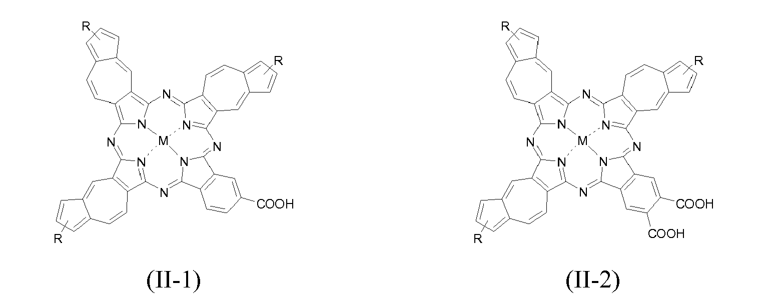 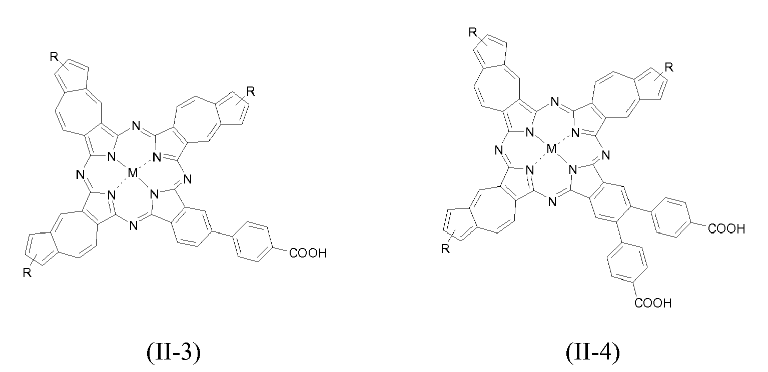 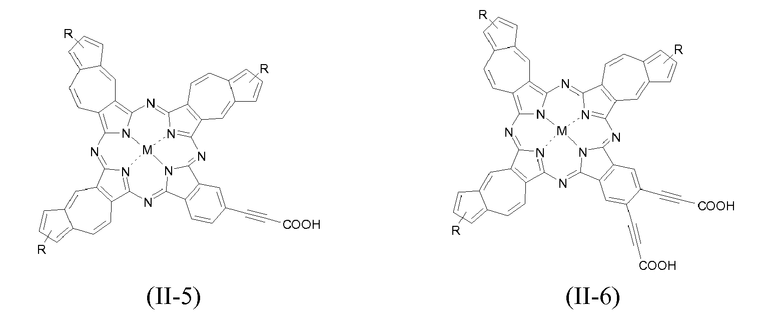 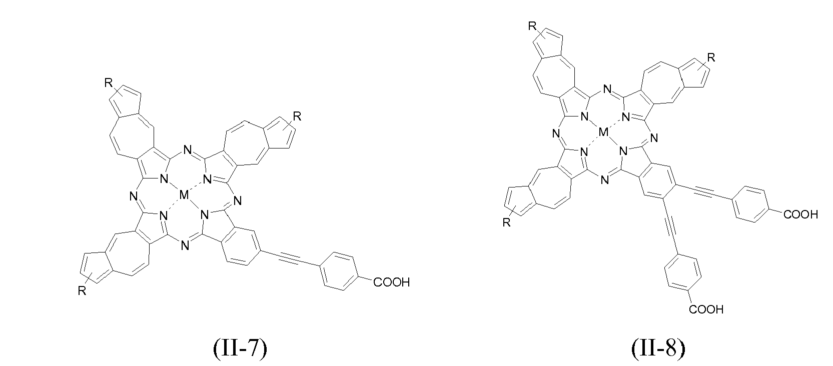 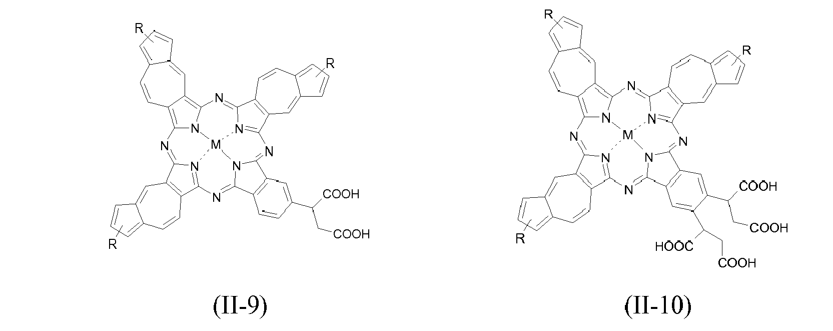 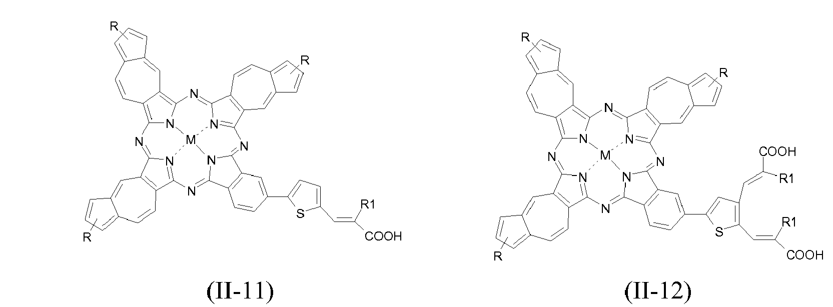 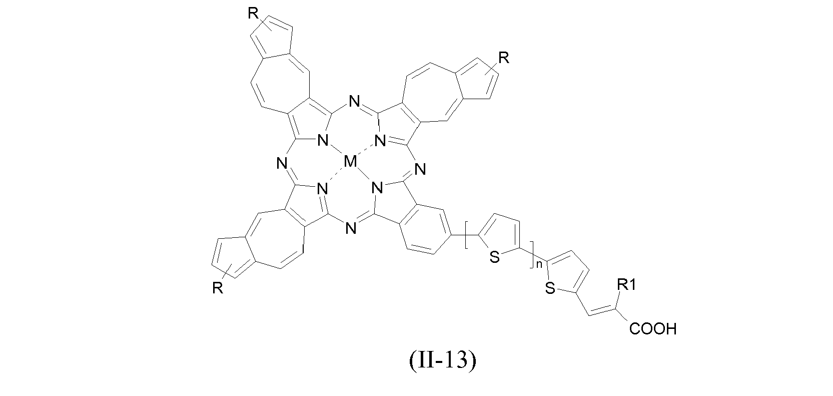 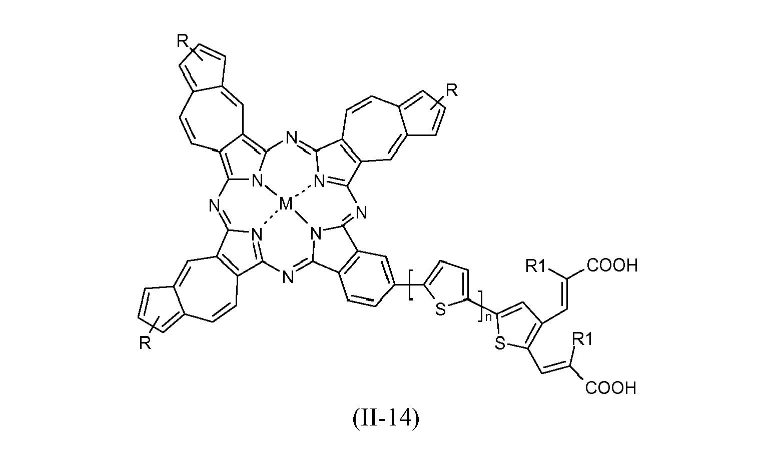 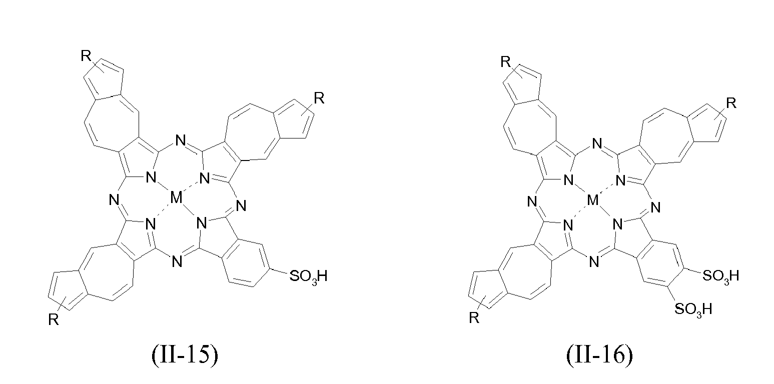 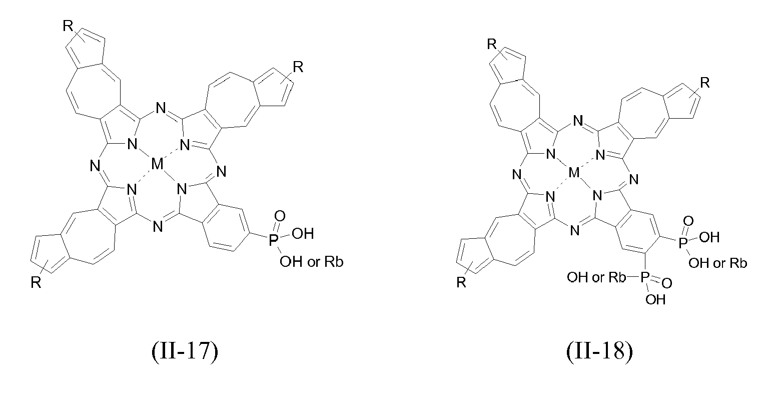 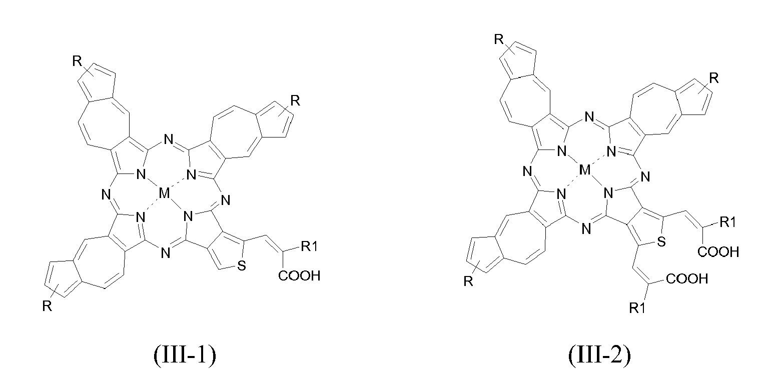 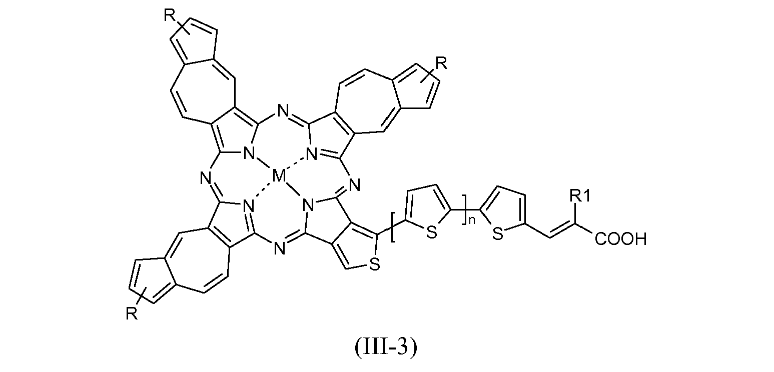 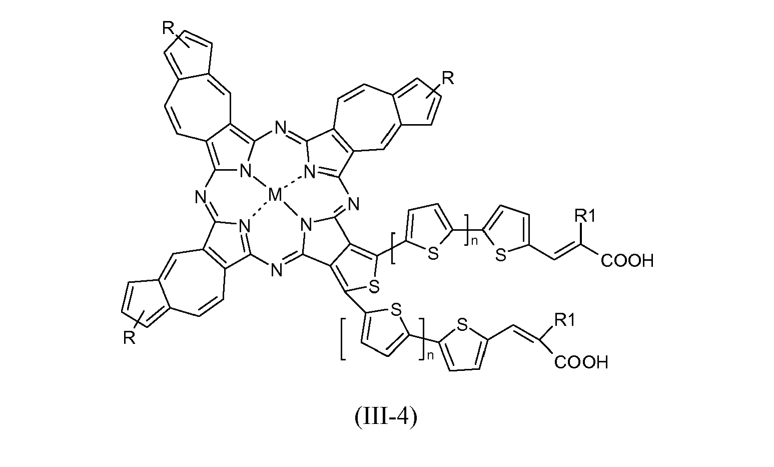 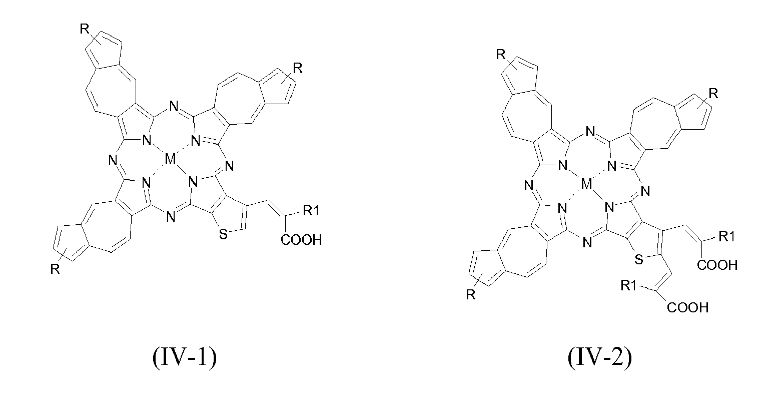 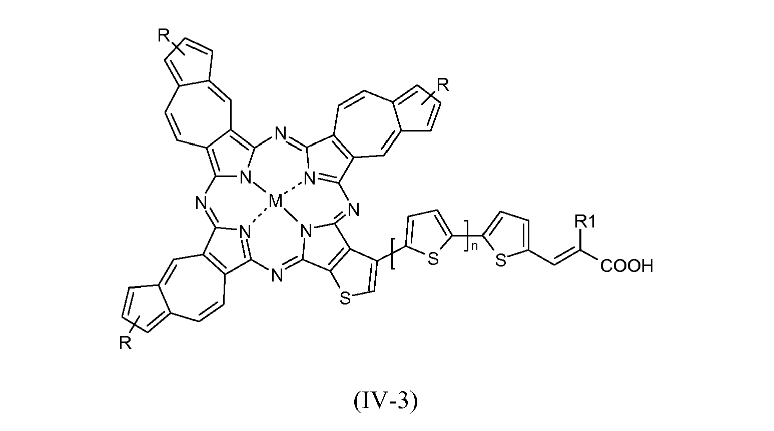 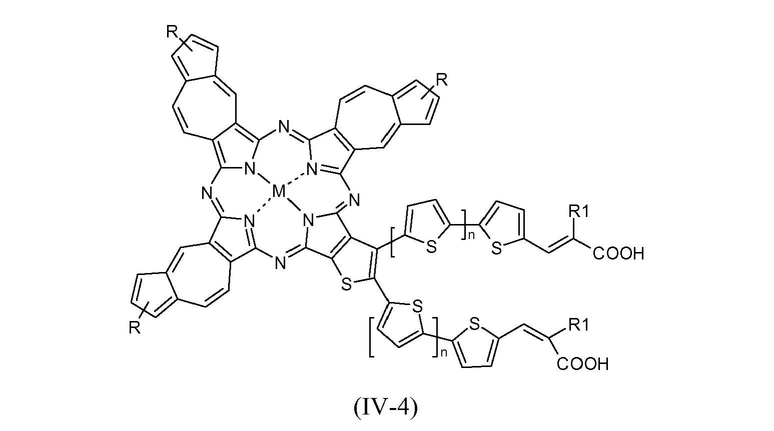 wherein M, R and Rb have the same meaning as denoted in the above, R1 is -CN, -COOH or optionally halogenated alkyl groups, and n is 0, 1, 2, 3, 4 or 5.

9. The azulenocyanine analogue compound according to any one of Claims 1 to 8, comprising at least one fluorine, preferably substituents R, R1 and/or Rb comprise at least one fluorine.

10. Use of the azulenocyanine analogue compound according to any one of Claims 1 to 9, as a dye, preferably in a dye-sensitized solar cell (DSSC) or a solid-state organic photovoltaic device (OPV).

11. A method for making the azulenocyanine analogue compound according to any one of Claims 1 to 9, comprising a step of reacting at least one azulene dinitrile and at least one other aromatic dinitrile different from the at least one azulene dinitrile.

12. The method according to Claim 11, further comprising a step of utilizing an M-containing precursor, preferably a metal salt or derivative of M, more preferably a Zn salt such as zinc acetate, magnesium oxide, an aluminum derivative such as aluminum chloride or bromide, a silicon derivative such as tetrachlorosilane, a stannous halide (SnHal₂), or a tin derivative such as tin dichloride (SnCl₂).

13. The method according to Claim 11 or 12, wherein the other aromatic dinitrile different from the at least one azulene dinitrile as building block is selected from the group consisting of : 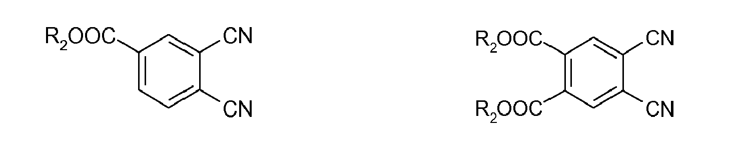 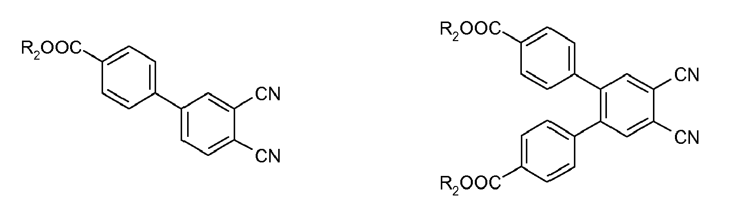 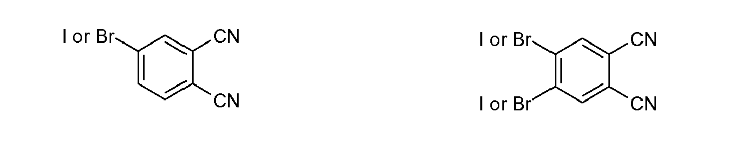 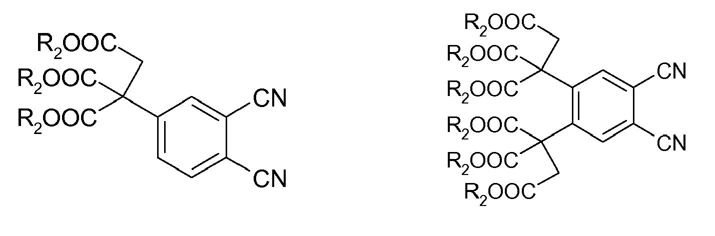 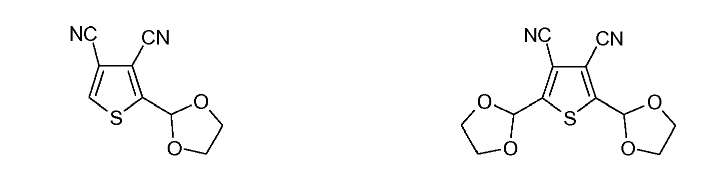 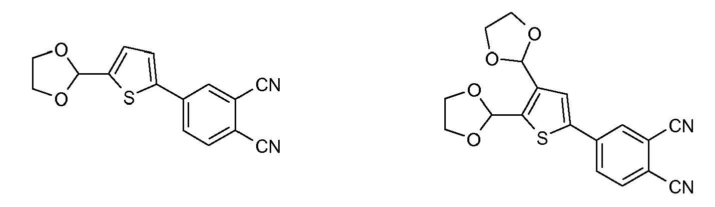 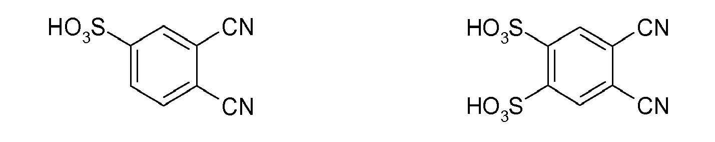 wherein R2 is selected from alkyl groups, preferably methyl or ethyl group.

14. A dye-sensitized solar cell comprising the azulenocyanine analogue compound according to any one of Claims 1 to 9.

15. The dye-sensitized solar cell according to Claim 14, comprising at least the fluorinated azulenocyanine analogue compound according to Claim 9 as a dye and at least TiOF₂ as semiconductor.
